# EUROPEAN PATENT APPLICATION

(11) **EP 4 623 828 A1**
(43) Date of publication of application: **01.10.2025**
(21) Application number: 25164775.6
(22) Date of filing: 19.03.2025
(51) Int. Cl.: A61B 6/02, A61B 6/00

(54) **IMAGE PROCESSING APPARATUS, IMAGE PROCESSING METHOD, AND IMAGE PROCESSING PROGRAM**

(30) Priority: 25.03.2024 JP 2024048733
(71) Applicant: FUJIFILM Corporation, Tokyo 106-8620 (JP)
(72) Inventor: MURASE, Takashi, Tokyo, 106-8620 (JP); MORI, Yoshiki, Tokyo, 106-8620 (JP)
(74) Representative: Dehns Germany Partnerschaft mbB

(57) **Abstract**

An image processing apparatus including a processor that: acquires projection data corresponding to a phase specified by an electrocardiogram of a subject, the projection data being imaged in synchronization with the electrocardiogram using radiation sequentially emitted from plural directions to an imaging portion of the subject, specifies a provisional stationary phase assumed to be a stationary phase of the subject based on a first reconstructed image reconstructed from the projection data corresponding to a phase included in a first phase range among the phases, generates a second reconstructed image in which a movement of the subject is corrected, the second reconstructed image being reconstructed from the projection data corresponding to a phase included in the first phase range and included in a second phase range including the provisional stationary phase, among the phases, and specifies the stationary phase of the subject based on the second reconstructed image.

## Description

### BACKGROUND

### Technical Field

The present disclosure relates to an image processing apparatus, an image processing method, and an image processing program.

### Related Art

In an examination using a radiation computed tomography (CT) apparatus, a technique of analyzing a periodic movement of a heart from electrocardiographic information of a subject obtained during imaging and specifying a stationary phase, which is a phase with the least amount of movement, is known (for example, see JP2003-204961A). Using the specified stationary phase, for example, imaging is performed in synchronization with the stationary phase, or an image for diagnosis is reconstructed using projection data in the stationary phase.

However, in the technique in the related art, in a case of attempting to detect the stationary phase with higher accuracy, a significant amount of time may be required for processing.

### SUMMARY

The present disclosure has been made in consideration of the above circumstances, and an object thereof is to provide an image processing apparatus, an image processing method, and an image processing program capable of accurately specifying a stationary phase while suppressing a time required for processing.

In order to achieve the above object, a first aspect of the present disclosure provides an image processing apparatus comprising: at least one processor, in which the processor acquires projection data corresponding to a phase specified by an electrocardiogram of a subject, the projection data being imaged in synchronization with the electrocardiogram using radiation sequentially emitted from a plurality of directions to an imaging portion of the subject, specifies a provisional stationary phase assumed to be a stationary phase of the subject based on a first reconstructed image reconstructed from the projection data corresponding to a phase included in a first phase range among the phases, generates a second reconstructed image in which a movement of the subject is corrected, the second reconstructed image being reconstructed from the projection data corresponding to a phase included in the first phase range and included in a second phase range including the provisional stationary phase, among the phases, and specifies the stationary phase of the subject based on the second reconstructed image.

A second aspect provides the image processing apparatus according to the first aspect, in which the processor generates the second reconstructed image under a first reconstruction condition, specifies a third phase range including the stationary phase of the subject from a phase corresponding to the second reconstructed image, and specifies the stationary phase of the subject based on a third reconstructed image that is reconstructed under a second reconstruction condition from the projection data corresponding to a phase included in the third phase range and in which a movement of the subject is corrected, and the second reconstruction condition is a processing condition for obtaining a reconstructed image of higher image quality than the first reconstruction condition.

A third aspect provides the image processing apparatus according to the second aspect, in which the processor specifies, as the third phase range, a range corresponding to a phase in which a movement of the subject is smaller than a predetermined threshold value.

A fourth aspect provides the image processing apparatus according to the first aspect, in which the processor generates the first reconstructed image at a first phase interval, generates the second reconstructed image at a second phase interval wider than the first phase interval, generates a third reconstructed image reconstructed from the projection data corresponding to a phase in which the second reconstructed image is generated, and specifies the stationary phase of the subject based on a deviation between the second reconstructed image and the third reconstructed image for each corresponding phase.

A fifth aspect provides the image processing apparatus according to the fourth aspect, in which the processor specifies, as the stationary phase of the subject, a phase in which the deviation between the second reconstructed image and the third reconstructed image is minimized.

A sixth aspect provides the image processing apparatus according to the first aspect, in which the first phase range is a range corresponding to at least one of a diastole or a systole of the subject.

In order to achieve the above object, a seventh aspect of the present disclosure provides an image processing method comprising: causing a processor included in an image processing apparatus to execute acquiring projection data corresponding to a phase specified by an electrocardiogram of a subject, the projection data being imaged in synchronization with the electrocardiogram using radiation sequentially emitted from a plurality of directions to an imaging portion of the subject, specifying a provisional stationary phase assumed to be a stationary phase of the subject based on a first reconstructed image reconstructed from the projection data corresponding to a phase included in a first phase range among the phases, generating a second reconstructed image in which a movement of the subject is corrected, the second reconstructed image being reconstructed from the projection data corresponding to a phase included in the first phase range and included in a second phase range including the provisional stationary phase, among the phases, and specifying the stationary phase of the subject based on the second reconstructed image.

In order to achieve the above object, an eighth aspect of the present disclosure provides an image processing program for causing a processor included in an image processing apparatus to execute a process comprising: acquiring projection data corresponding to a phase specified by an electrocardiogram of a subject, the projection data being imaged in synchronization with the electrocardiogram using radiation sequentially emitted from a plurality of directions to an imaging portion of the subject; specifying a provisional stationary phase assumed to be a stationary phase of the subject based on a first reconstructed image reconstructed from the projection data corresponding to a phase included in a first phase range among the phases; generating a second reconstructed image in which a movement of the subject is corrected, the second reconstructed image being reconstructed from the projection data corresponding to a phase included in the first phase range and included in a second phase range including the provisional stationary phase, among the phases; and specifying the stationary phase of the subject based on the second reconstructed image.

According to the present disclosure, it is possible to accurately specify the stationary phase while suppressing the time required for the processing.

### BRIEF DESCRIPTION OF THE DRAWINGS

Fig. 1 is a configuration diagram showing an example of a configuration of a radiation CT imaging apparatus of an embodiment.
Fig. 2 is a configuration diagram showing an example of a configuration of a console of the embodiment.
Fig. 3 is a functional block diagram showing an example of a function of the console of the embodiment.
Fig. 4A is a diagram schematically showing an example of an image of a cardiac phase.
Fig. 4B is a diagram for describing a method of specifying a provisional optimal cardiac phase (optimal cardiac phase in CardioHarmony (registered trademark)).
Fig. 5 is a flowchart showing an example of a flow of image processing of the embodiment.

### DESCRIPTION OF EMBODIMENTS

Hereinafter, embodiments of the present invention will be described in detail with reference to the drawings. The present embodiment does not limit the present invention.

### First Embodiment

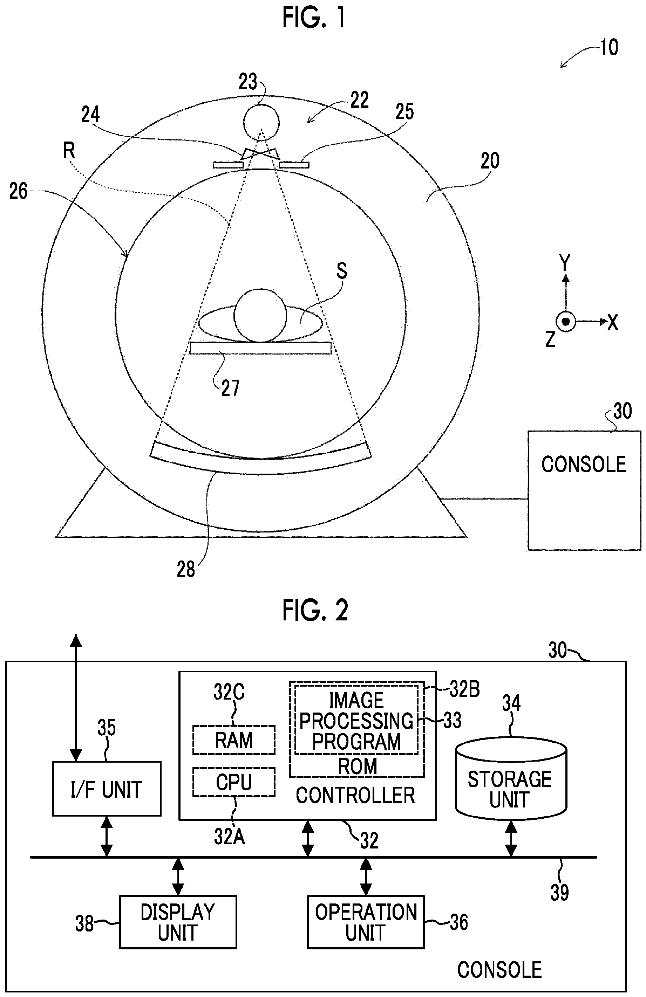

First, an example of a configuration of a radiation computed tomography (CT) imaging apparatus of the present embodiment will be described. Fig. 1 is a configuration diagram showing an example of a configuration of a radiation CT imaging apparatus 10 of the present embodiment.

As shown in Fig. 1, the radiation CT imaging apparatus of the present embodiment comprises a gantry 20, a couch 27, and a console 30. In the following description, a lateral direction in Fig. 1 is defined as an X axis, a longitudinal direction is defined as a Y axis, and a direction orthogonal to an XY plane is defined as a Z axis.

The gantry 20 has an opening portion 26, and a subject S to be imaged is disposed in the opening portion 26 in a state of being placed on a couch 27. The gantry 20 and the couch 27 can be relatively moved in a Z axis direction.

Inside the gantry 20, a radiation generation device 23 having a radiation tube (not shown), a bow tie filter 24, and a collimator 25, and a detector panel 28 are disposed in a state of facing each other across the subject S. Radiation R emitted from the radiation generation device 23 is formed into a beam shape suitable for a size of the subject S by the bow tie filter 24 and the collimator 25 and is emitted to the subject S. The detector panel 28 detects radiation transmitted through the subject S and generates projection data according to a dose of the detected radiation.

The radiation generation device 23 and the detector panel 28 are rotated around the subject S by a rotation driving unit (not shown) of the gantry 20. The radiation irradiation from the radiation generation device 23 and the radiation detection of the detector panel 28 are repeated while both the radiation generation device 23 and the detector panel 28 are rotated, thereby acquiring projection data at various projection angles. A plurality of projection data acquired by the detector panel 28 are output to the console 30.

A dose of the radiation emitted from the radiation generation device 23, a rotation speed of the gantry 20, a relative movement speed between the gantry 20 and the couch 27, and the like are set by the console 30 based on a scan condition input by a user such as a technician. In a case of electrocardiogram (ECG) synchronization imaging, imaging is performed in synchronization with a phase (cardiac phase) of a movement of a heart based on information on an electrocardiograph (not shown) attached to the subject S, and, in a case of asynchronous imaging, imaging is performed under automatic exposure control.

The console 30 of the present embodiment performs control related to the acquisition of the projection data, specification of a stationary phase, generation of a medical image, and the like. The console 30 of the present embodiment is an example of an image processing apparatus according to the present disclosure. The console 30 of the present embodiment is, for example, a server computer.

As shown in Fig. 2, the console 30 comprises a controller 32, a storage unit 34, an interface (I/F) unit 35, an operation unit 36, and a display unit 38. The controller 32, the storage unit 34, the I/F unit 35, the operation unit 36, and the display unit 38 are connected to each other via a bus 39 such as a system bus or a control bus so as to be able to transmit and receive various types of information.

The controller 32 of the present embodiment controls the overall operation of the console 30. The controller 32 comprises a central processing unit (CPU) 32A, a read only memory (ROM) 32B, and a random access memory (RAM) 32C. The ROM 32B stores in advance various programs including an image processing program 33 to be described below, which is executed by the CPU 32A. The RAM 32C temporarily stores various types of data.

The storage unit 34 stores the projection data output from the detector panel 28, various other types of information, and the like. Specific examples of the storage unit 34 include a storage medium such as a hard disk drive (HDD), a solid state drive (SSD), and a flash memory.

The I/F unit 35 performs communication of various types of information with a rotation driving unit (not shown) of the gantry 20, the radiation generation device 23, and the detector panel 28 through wired communication or wireless communication. The console 30 of the present embodiment receives the projection data from the detector panel 28 via the I/F unit 35. The received projection data is stored in the storage unit 34 in a state of being associated with the cardiac phase.

The console 30 acquires a plurality of projection data from the detector panel 28 via the I/F unit 35. The controller 32 performs reconstruction processing on the acquired plurality of projection data to generate a tomographic image of the subject S.

The operation unit 36 is used by the user to input a scan condition for acquiring the projection data, an instruction or various types of information related to the generation and display of the image, and the like. The operation unit 36 is not particularly limited, and examples thereof include various switches, buttons, a touch panel, a touch pen, a keyboard, and a mouse. The display unit 38 displays various types of information, a medical image, and the like. The operation unit 36 and the display unit 38 may be integrated into a touch panel display. In addition, for example, the operation unit 36 may receive a voice input from the user.

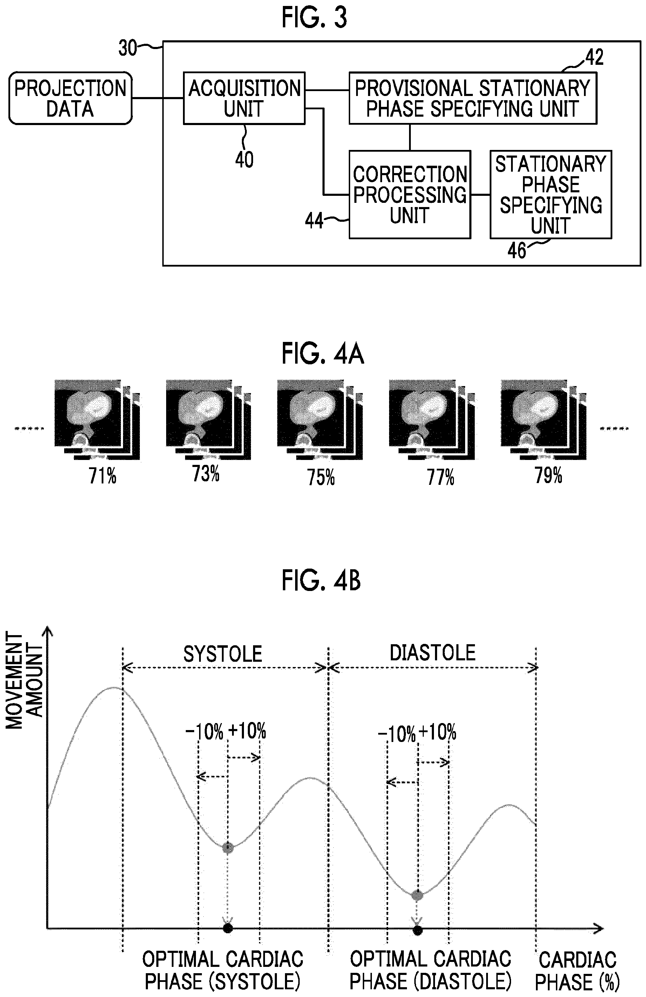

Fig. 3 shows a functional block diagram showing an example of a function of the console 30. The console 30 comprises an acquisition unit 40, a provisional stationary phase specifying unit 42, a correction processing unit 44, and a stationary phase specifying unit 46. As an example, in the console 30 of the present embodiment, the CPU 32A of the controller 32 executes the image processing program 33, so that the CPU 32A functions as the acquisition unit 40, the provisional stationary phase specifying unit 42, the correction processing unit 44, and the stationary phase specifying unit 46.

The acquisition unit 40 has a function of acquiring the projection data from the detector panel 28. Specifically, as described above, the acquisition unit 40 acquires projection data corresponding to a phase specified by an electrocardiogram of the subject S, the projection data being imaged in synchronization with the electrocardiogram using radiation sequentially emitted from a plurality of directions to the subject S, from the detector panel 28 via the I/F unit 35. The acquisition unit 40 may acquire the projection data that has been acquired in advance from the detector panel 28 and stored in the storage unit 34 in association with the cardiac phase, from the storage unit 34. The acquisition unit 40 outputs the acquired projection data to the provisional stationary phase specifying unit 42 and the correction processing unit 44.

The provisional stationary phase specifying unit 42 has a function of specifying a provisional stationary phase assumed to be a stationary phase based on a first reconstructed image reconstructed from the projection data corresponding to a phase included in each of a diastole and a systole of the heart among the phases. The stationary phase refers to a phase in which a movement of the heart is minimized among all the phases or a phase close to resting. In addition, the stationary phase may be referred to as an optimal cardiac phase. Each of the diastole and the systole of the heart of the present embodiment is an example of a first phase range of the present disclosure. In addition, the first reconstructed image reconstructed by the provisional stationary phase specifying unit 42 is an example of a first reconstructed image of the present disclosure.

A method by which the provisional stationary phase specifying unit 42 specifies the provisional stationary phase is not limited. For example, a phase detected as the optimal cardiac phase using CardioHarmony (registered trademark), which is an automatic phase search technique, may be specified as the provisional stationary phase. CardioHarmony (registered trademark) is a technique of extracting the amount of the movement of the entire heart from an image (reconstructed images) created for each cardiac phase and detecting a phase in which the amount is minimized as the optimal cardiac phase. Specifically, first, for the entire heart, as shown in Fig. 4A, images of cardiac phases of 0% to 99% are generated. Then, the movement amount is extracted at each phase from a contrast part of the created image. Furthermore, as shown in a graph of Fig. 4B, a correspondence relationship between the movement amount and the phase is specified, and the phase in which the movement amount is minimized is detected as the optimal cardiac phase. The optimal cardiac phase is detected for each of the diastole and the systole of the heart. That is, two optimal cardiac phases are detected in one cycle of expansion and contraction of the heart. In addition, for example, a technique disclosed in JP4157302B may be used.

The provisional stationary phase specifying unit 42 outputs the specified provisional stationary phase to the correction processing unit 44.

The correction processing unit 44 has a function of generating a second reconstructed image in which a movement of the heart is corrected, the second reconstructed image being reconstructed from the projection data corresponding to a phase included in each of the diastole and the systole of the heart and included in a phase range including the provisional stationary phase, among the phases. As an example, the correction processing unit 44 of the present embodiment generates the second reconstructed image by reconstructing the projection data corresponding to a phase included in a range of ±10% of the provisional stationary phase (see Fig. 4B) while correcting the movement of the heart during the imaging. As a specific example, in a case in which the provisional stationary phase in the diastole is 77%, the projection data corresponding to a phase included in a phase range of 67% or more and 87% or less is used. In addition, in a case in which the provisional stationary phase in the systole is 44%, the projection data corresponding to a phase included in a phase range of 34% or more and 54% or less is used. The range of ±10% of the provisional stationary phase in the present embodiment is an example of a second phase range of the present disclosure.

A method by which the correction processing unit 44 corrects the movement of the heart, in other words, a method by which the correction processing unit 44 reconstructs the projection data while correcting the movement of the heart is not limited. For example, the second reconstructed image in which the movement of the heart is corrected may be generated by acquiring movement information of the heart from an image and reconstructing the projection data using a ratio of tube currents used for capturing the image and the movement information.

A first image and a second image of confronting positions are generated using the projection data corresponding to a phase included in the range of ±10% of the provisional stationary phase, noise reduction processing and post-noise reduction registration processing are performed on each of the first image and the second image, and the movement information of the subject is acquired. In this case, the second reconstructed image may be generated by adjusting at least one of a type or a parameter of a noise reduction filter used for the noise reduction processing and a parameter of a non-rigid registration algorithm used for the registration processing based on a ratio of tube currents in a case in which the first image and the second image are acquired, and reconstructing the projection data using the movement information. The reconstructed image reconstructed by the stationary phase specifying unit 46 is an example of a second reconstructed image of the present disclosure.

The second reconstructed image generated by the correction processing unit 44 is output to the stationary phase specifying unit 46.

The stationary phase specifying unit 46 has a function of specifying a stationary phase of the heart based on the second reconstructed image. A method by which the stationary phase specifying unit 46 specifies the stationary phase is not limited. For example, a phase detected as the optimal cardiac phase using CardioHarmony (registered trademark) described above may be specified as the stationary phase. The stationary phase specifying unit 46 of the present embodiment specifies the stationary phase in the diastole and the stationary phase in the systole of the heart.

Next, an action of the console 30 of the present embodiment will be described.

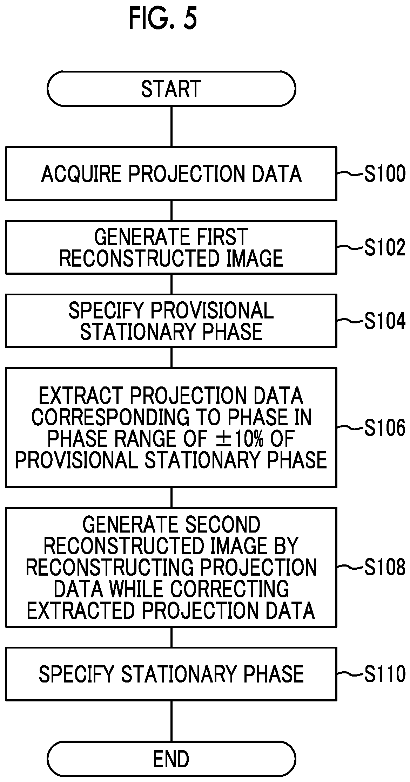

As an example, in a case in which the console 30 of the present embodiment receives display conditions for a radiation image X and an ultrasound image U to be displayed, which are input by the user using the operation unit 36, are received, the CPU 32A of the controller 32 executes the image processing program 33 stored in the ROM 32B to execute image processing shown as an example in Fig. 5. Fig. 5 is a flowchart showing an example of a flow of the image processing in the console 30 of the present embodiment.

First, in step S100 of Fig. 5, the acquisition unit 40 acquires projection data associated with a cardiac phase, which is obtained by imaging the heart of the subject S using the radiation CT imaging apparatus 10, as described above.

In next step S102, the provisional stationary phase specifying unit 42 generates a first reconstructed image as described above. The projection data corresponding to a phase included in each of a diastole and a systole of the heart (see Fig. 4B) among the phases is reconstructed to generate a first reconstructed image.

In next step S104, the provisional stationary phase specifying unit 42 specifies a provisional stationary phase by using the first reconstructed image generated in step S102 as described above.

In a case in which the provisional stationary phase specifying unit 42 uses CardioHarmony (registered trademark), the processes of step S102 and step S104 are performed together.

In next step S106, the correction processing unit 44 extracts the projection data corresponding to a phase in a phase range of ±10% (see Fig. 4B) of the provisional stationary phase specified in step S104, from the projection data acquired in step S100, as described above.

In next step S108, the correction processing unit 44 generates a second reconstructed image by reconstructing the projection data extracted in step S106 while correcting the movement of the heart during the imaging, as described above.

In next step S110, the stationary phase specifying unit 46 specifies the stationary phase based on the second reconstructed image generated in step S108. The stationary phase specified here is output to a predetermined output destination. For example, in a case of outputting the stationary phase to the display unit 38 of the console 30, information indicating the stationary phase and the second reconstructed image in the stationary phase may be displayed on the display unit 38. In addition, in a case of outputting the stationary phase to the storage unit 34, the stationary phase may be stored in the storage unit 34 in association with at least one of the projection data acquired by the acquisition unit 40 or the second reconstructed image in the stationary phase. In a case in which the process of step S110 ends, the image processing shown in Fig. 5 ends.

As described above, with the console 30 of the present embodiment, the provisional stationary phase is specified using a method that has a relatively small processing load and does not require a processing time, and a reconstructed image in which the movement of the heart is corrected is generated for a phase in a phase range narrowed down by the specified provisional phase. Then, the stationary phase is specified from the reconstructed image in which the movement of the heart is corrected. As a result, with the console 30 of the present embodiment, it is possible to accurately specify the stationary phase while suppressing the time required for the processing.

### Second Embodiment

In the present embodiment, since the processes performed by the correction processing unit 44 and the stationary phase specifying unit 46 are different, the different processes will be described.

In a case of generating the second reconstructed image in which the movement of the subject is corrected as described above, the correction processing unit 44 of the present embodiment generates the second reconstructed image under a first reconstruction condition. Then, the correction processing unit 44 specifies a third phase range including the stationary phase of the subject from a phase corresponding to the second reconstructed image. This third phase range is a phase range in which the movement of the heart is smaller than a predetermined threshold value. The third phase range is a range that is equal to or smaller than a phase range of ±10% of the provisional stationary phase, and is a range included in the phase range of ±10% of the provisional stationary phase.

In addition, the correction processing unit 44 generates a third reconstructed image in which the movement of the subject is corrected, the third reconstructed image being reconstructed under a second reconstruction condition from the projection data corresponding to a phase included in a phase range of ±5% of the newly specified provisional phase range.

Here, the second reconstruction condition is a condition under which image quality of the reconstructed image obtained by the reconstruction is higher than that under the first reconstruction condition. In this case, since a processing load for obtaining a high-quality image is large, the time required for processing is relatively long. Examples of the reconstruction condition in this case include a condition related to a slice thickness or a slice interval.

On the other hand, the first reconstruction condition is a condition under which it is sufficient to obtain image quality sufficient for phase detection, and which has lower image quality than the reconstructed image obtained under the second reconstruction condition. In this case, the image quality can be suppressed, so that the time required for processing can be shortened. Examples of the reconstruction condition in this case include a condition related to a field of view (FOV), a filter, and a matrix size.

Furthermore, the correction processing unit 44 specifies the stationary phase of the heart as described above based on the generated third reconstructed image.

As described above, in the present embodiment, even in a case in which a corrected image in which the movement of the heart is corrected is reconstructed, the phase range is further narrowed down by a method that requires relatively little processing time, and the reconstruction is performed for the narrowed-down phase range with higher image quality. Therefore, with the console 30 of the present embodiment, it is possible to specify the stationary phase with higher accuracy while suppressing an increase in processing time.

Unlike the present embodiment, the process of the provisional stationary phase specifying unit 42 may be omitted. That is, the processes of the correction processing unit 44 and the stationary phase specifying unit 46 of the present embodiment may be executed for the entire phase range.

### Third Embodiment

In the present embodiment, since the processes performed by the provisional stationary phase specifying unit 42, the correction processing unit 44, and the stationary phase specifying unit 46 are different, the different processes will be described.

The provisional stationary phase specifying unit 42 generates the first reconstructed image at a first phase interval.

The correction processing unit 44 generates the second reconstructed image at a second phase interval wider than the first phase interval.

In addition, the correction processing unit 44 generates a third reconstructed image reconstructed from the projection data corresponding to a phase in which the second reconstructed image is generated. The third reconstructed image is a reconstructed image in which the movement of the heart is not corrected.

Then, the stationary phase specifying unit 46 specifies the stationary phase of the heart based on a deviation between the second reconstructed image and the third reconstructed image for each corresponding phase. In this case, the stationary phase specifying unit 46 may specify, as the stationary phase of the heart, a phase in which the deviation between the second reconstructed image and the third reconstructed image is minimized.

As described above, in the present embodiment, the stationary phase is specified while comparing the second reconstructed image in which the movement is corrected with the third reconstructed image in which the movement is not corrected. As a result, with the console 30 of the present embodiment, the stationary phase can be specified by relatively easy processing.

As described above, with the console 30 according to each of the above-described embodiments, it is possible to accurately specify the stationary phase while suppressing the time required for the processing.

In each of the above-described embodiments, as a hardware structure of a processing unit that executes various types of processing, such as the acquisition unit 40, the provisional stationary phase specifying unit 42, the correction processing unit 44, and the stationary phase specifying unit 46, various processors shown below can be used. As described above, the various processors include a programmable logic device (PLD) as a processor of which the circuit configuration can be changed after manufacture, such as a field programmable gate array (FPGA), a dedicated electrical circuit as a processor having a dedicated circuit configuration for executing specific processing such as an application specific integrated circuit (ASIC), and the like, in addition to the CPU as a general-purpose processor that functions as various processing units by executing software (program).

One processing unit may be configured of one of the various processors, or may be configured of a combination of the same or different types of two or more processors (for example, a combination of a plurality of FPGAs or a combination of the CPU and the FPGA). In addition, a plurality of processing units may be configured of one processor.

As an example in which a plurality of processing units are configured of one processor, first, as typified by a computer such as a client or a server, one processor is configured of a combination of one or more CPUs and software, and this processor functions as a plurality of processing units. Second, as typified by a system on chip (SoC) or the like, a processor that realizes the functions of the entire system including the plurality of processing units by using one integrated circuit (IC) chip is used. As described above, various processing units are configured by using one or more of the various processors as a hardware structure.

Furthermore, as the hardware structure of these various processors, more specifically, an electric circuit (circuitry) in which circuit elements such as semiconductor elements are combined can be used.

In addition, in the above-described embodiment, the aspect has been described in which the image processing program 33 is stored (installed) in the storage unit 34 of the console 30 in advance, but the present invention is not limited to this. The image processing program 33 may be provided in an aspect in which the image processing program 33 is recorded in a recording medium, such as a compact disc read only memory (CD-ROM), a digital versatile disc read only memory (DVD-ROM), and a universal serial bus (USB) memory. In addition, the image processing program 33 may be downloaded from an external device via a network.

In addition, the configurations and operations of the radiation CT imaging apparatus 10, the console 30, and the like described in each of the above-described embodiments are merely examples, and it is needless to say that they can be changed according to the situation without departing from the gist of the present invention. In addition, it is needless to say that the above-described embodiments may be combined as appropriate.

Regarding the above-described embodiments, the following appendices are further disclosed.

### Appendix 1

An image processing apparatus comprising:
at least one processor,
in which the processor
   acquires projection data corresponding to a phase specified by an electrocardiogram of a subject, the projection data being imaged in synchronization with the electrocardiogram using radiation sequentially emitted from a plurality of directions to an imaging portion of the subject,
   specifies a provisional stationary phase assumed to be a stationary phase of the subject based on a first reconstructed image reconstructed from the projection data corresponding to a phase included in a first phase range among the phases,
   generates a second reconstructed image in which a movement of the subject is corrected, the second reconstructed image being reconstructed from the projection data corresponding to a phase included in the first phase range and included in a second phase range including the provisional stationary phase, among the phases, and
   specifies the stationary phase of the subject based on the second reconstructed image.

### Appendix 2

The image processing apparatus according to Appendix 1,
in which the processor
   generates the second reconstructed image under a first reconstruction condition,
   specifies a third phase range including the stationary phase of the subject from a phase corresponding to the second reconstructed image, and
   specifies the stationary phase of the subject based on a third reconstructed image that is reconstructed under a second reconstruction condition from the projection data corresponding to a phase included in the third phase range and in which a movement of the subject is corrected, and
the second reconstruction condition is a processing condition for obtaining a reconstructed image of higher image quality than the first reconstruction condition.

### Appendix 3

The image processing apparatus according to Appendix 2,
in which the processor specifies, as the third phase range, a range corresponding to a phase in which a movement of the subject is smaller than a predetermined threshold value.

### Appendix 4

The image processing apparatus according to Appendix 1,
in which the processor
generates the first reconstructed image at a first phase interval,
generates the second reconstructed image at a second phase interval wider than the first phase interval,
generates a third reconstructed image reconstructed from the projection data corresponding to a phase in which the second reconstructed image is generated, and
specifies the stationary phase of the subject based on a deviation between the second reconstructed image and the third reconstructed image for each corresponding phase.

### Appendix 5

The image processing apparatus according to Appendix 4,
in which the processor specifies, as the stationary phase of the subject, a phase in which the deviation between the second reconstructed image and the third reconstructed image is minimized.

### Appendix 6

The image processing apparatus according to any one of Appendices 1 to 5,
in which the first phase range is a range corresponding to at least one of a diastole or a systole of the subject.

### Appendix 7

An image processing method comprising:
causing a processor included in an image processing apparatus to execute
acquiring projection data corresponding to a phase specified by an electrocardiogram of a subject, the projection data being imaged in synchronization with the electrocardiogram using radiation sequentially emitted from a plurality of directions to an imaging portion of the subject,
specifying a provisional stationary phase assumed to be a stationary phase of the subject based on a first reconstructed image reconstructed from the projection data corresponding to a phase included in a first phase range among the phases,
generating a second reconstructed image in which a movement of the subject is corrected, the second reconstructed image being reconstructed from the projection data corresponding to a phase included in the first phase range and included in a second phase range including the provisional stationary phase, among the phases, and
specifying the stationary phase of the subject based on the second reconstructed image.

### Appendix 8

An image processing program for causing a processor included in an image processing apparatus to execute a process comprising:
acquiring projection data corresponding to a phase specified by an electrocardiogram of a subject, the projection data being imaged in synchronization with the electrocardiogram using radiation sequentially emitted from a plurality of directions to an imaging portion of the subject;
specifying a provisional stationary phase assumed to be a stationary phase of the subject based on a first reconstructed image reconstructed from the projection data corresponding to a phase included in a first phase range among the phases;
generating a second reconstructed image in which a movement of the subject is corrected, the second reconstructed image being reconstructed from the projection data corresponding to a phase included in the first phase range and included in a second phase range including the provisional stationary phase, among the phases; and
specifying the stationary phase of the subject based on the second reconstructed image.

### Explanation of References

10: radiation CT imaging apparatus
20: gantry
23: radiation generation device
24: bow tie filter
25: collimator
26: opening portion
27: couch
28: detector panel
30: console
32: controller
32A: CPU
32B: ROM
32C: RAM
33: image processing program
34: storage unit
35: I/F unit
36: operation unit
38: display unit
39: bus
40: acquisition unit
42: provisional stationary phase specifying unit
44: correction processing unit
46: stationary phase specifying unit
R: radiation
S: subject

## Claims

1. An image processing apparatus comprising:
at least one processor, wherein the processor:
acquires projection data corresponding to a phase specified by an electrocardiogram of a subject, the projection data being imaged in synchronization with the electrocardiogram using radiation sequentially emitted from a plurality of directions to an imaging portion of the subject;
specifies a provisional stationary phase assumed to be a stationary phase of the subject based on a first reconstructed image reconstructed from the projection data corresponding to a phase included in a first phase range among the phases;
generates a second reconstructed image in which a movement of the subject is corrected, the second reconstructed image being reconstructed from the projection data corresponding to a phase included in the first phase range and included in a second phase range including the provisional stationary phase, among the phases; and
specifies the stationary phase of the subject based on the second reconstructed image.

2. The image processing apparatus according to claim 1, wherein the processor:
generates the second reconstructed image under a first reconstruction condition,
specifies a third phase range including the stationary phase of the subject from a phase corresponding to the second reconstructed image, and
specifies the stationary phase of the subject based on a third reconstructed image that is reconstructed under a second reconstruction condition from the projection data corresponding to a phase included in the third phase range and in which a movement of the subject is corrected,
wherein the second reconstruction condition is a processing condition for obtaining a reconstructed image of higher image quality than the first reconstruction condition.

3. The image processing apparatus according to claim 2, wherein the processor specifies, as the third phase range, a range corresponding to a phase in which a movement of the subject is smaller than a predetermined threshold value.

4. The image processing apparatus according to any one of the preceding claims, wherein the processor:
generates the first reconstructed image at a first phase interval,
generates the second reconstructed image at a second phase interval wider than the first phase interval,
generates a third reconstructed image reconstructed from the projection data corresponding to a phase in which the second reconstructed image is generated, and
specifies the stationary phase of the subject based on a deviation between the second reconstructed image and the third reconstructed image for each corresponding phase.

5. The image processing apparatus according to claim 4, wherein the processor specifies, as the stationary phase of the subject, a phase in which the deviation between the second reconstructed image and the third reconstructed image is minimized.

6. The image processing apparatus according to any one of the preceding claims, wherein the first phase range is a range corresponding to at least one of a diastole or a systole of the subject.

7. An image processing method comprising:
causing a processor included in an image processing apparatus to execute:
acquiring projection data corresponding to a phase specified by an electrocardiogram of a subject, the projection data being imaged in synchronization with the electrocardiogram using radiation sequentially emitted from a plurality of directions to an imaging portion of the subject;
specifying a provisional stationary phase assumed to be a stationary phase of the subject based on a first reconstructed image reconstructed from the projection data corresponding to a phase included in a first phase range among the phases;
generating a second reconstructed image in which a movement of the subject is corrected, the second reconstructed image being reconstructed from the projection data corresponding to a phase included in the first phase range and included in a second phase range including the provisional stationary phase, among the phases; and
specifying the stationary phase of the subject based on the second reconstructed image.

8. A computer readable medium storing an image processing program for causing a processor included in an image processing apparatus to execute a process comprising:
acquiring projection data corresponding to a phase specified by an electrocardiogram of a subject, the projection data being imaged in synchronization with the electrocardiogram using radiation sequentially emitted from a plurality of directions to an imaging portion of the subject;
specifying a provisional stationary phase assumed to be a stationary phase of the subject based on a first reconstructed image reconstructed from the projection data corresponding to a phase included in a first phase range among the phases;
generating a second reconstructed image in which a movement of the subject is corrected, the second reconstructed image being reconstructed from the projection data corresponding to a phase included in the first phase range and included in a second phase range including the provisional stationary phase, among the phases; and
specifying the stationary phase of the subject based on the second reconstructed image.
